## Europäisches Patentamt

(19) **European Patent Office**

**Office européen des brevets**

(11) Veröffentlichungsnummer: **0 142 125**
**A2**

(12) **EUROPÄISCHE PATENTANMELDUNG**

(21) Anmeldenummer: **84113405.9**

(22) Anmeldetag: **07.11.84**

(51) Int. Cl.⁴: **C 07 K 17/02**
**A 61 K 37/14**

(30) Priorität: **10.11.83 DE 3340592**

(43) Veröffentlichungstag der Anmeldung:
**22.05.85 Patentblatt 85/21**

(84) Benannte Vertragsstaaten:
**AT BE CH DE FR GB IT LI LU NL SE**

(71) Anmelder: **Intermedicat GmbH**
**Gerliswilstrasse 45**
**CH-6020 Emmenbrücke(CH)**

(72) Erfinder:
**Die Erfinder haben auf ihre Nennung verzichtet**

(74) Vertreter: **von Kreisler, Alek, Dipl.-Chem. et al,**
**Deichmannhaus am Hauptbahnhof**
**D-5000 Köln 1(DE)**

(54) Konjugate makromolekularer Verbindungen an Hämoglobin, Verfahren zu ihrer Herstellung und sie enthaltende Arzneimittel.

(57) Die Erfindung betrifft Konjugate makromolekularer Verbindungen an Hämoglobin, enthaltend als Makromolekül ein Addukt eines oder mehrerer wasserlöslicher, ungeladener, physiologisch unbedenklicher Polymerer an einen anionischen Liganden sowie gereinigtes, von endogenen Liganden isoliertes Human-Hämoglobin A, wobei die Makromoleküle eine den Anforderungen entsprechend variable Molekulargewichtsverteilung von 400 bis 500.000 D aufweisen und nichtkovalent reversibel über die Liganden an das allosterische Bindungszentrum des Hämoglobins gebunden sind, Verfahren zur Herstellung derartiger Konjugate und sie enthaltende Arzneimittel.

Croydon Printing Company Ltd.

## Konjugate makromolekularer Verbindungen an Hämoglobin, Verfahren zu ihrer Herstellung und sie enthaltende Arzneimittel

Die Erfindung betrifft Konjugate makromolekularer Verbindungen an Hämoglobin, enthaltend als Makromolekül ein Addukt eines oder mehrerer wasserlöslicher, physiologisch unbedenklicher Polymerer an einen anionischen Liganden sowie gereinigtes, von endogenen Liganden isoliertes Humanhämoglobin A, wobei die Makromoleküle eine den Anforderungen entsprechend variable Molekulargewichtsverteilung von 400 bis 500.000 D aufweisen und nichtkovalent reversibel über die Liganden an das allosterische Bindungszentrum des Hämoglobins gebunden sind, ein Verfahren zur Herstellung derartiger Konjugate sowie Arzneimittel, die derartige Konjugate makromolekularer Verbindungen an Hämoglobin enthalten.

Durch Operationen und/oder Verletzungen bedingte Blut- bzw. Plasmaverluste des menschlichen Körpers können durch Infusionen von Vollblut oder, wegen der mit der Infusion von Vollblut verbundenen bekannten Risiken, auch durch die Infusion von Blutersatzstoffen oder Blutplasma-Streckmitteln, die zum Teil auch Sauerstoff übertragende Funktionen erfüllen, ausgeglichen werden.

Zur Zeit werden im wesentlichen zwei unterschiedliche Ansätze zur Entwicklung Sauerstoff übertragender Blutersatzflüssigkeiten beschrieben.

Beispielsweise werden in Med. Welt 32, 1338 (1981) und Med. Lab. Sci. 39, 45 (1982) Fluorokarbone als Blutgas transportierende Blutersatzmittel beschrieben. Der bedeutsamste Nachteil der Fluorokarbone, die in Form von Emulsionen vorliegen, besteht darin, daß sie weder aus dem Organismus vollständig ausgeschieden noch abgebaut werden, sondern sich in Organen wie der Leber oder Milz anreichern und selbst nach mehreren Jahren noch nachweisbar sein können. Einer breiten Anwendung steht auch entgegen, daß zur Atmung eine sauerstoffreiche Atmosphäre erforderlich ist. Außerdem treten offenbar Lagerungsprobleme auf.

Der zweite Ansatz, nämlich hämoglobinhaltige Lösungen als Blutersatzstoff oder Blutplasma-Streckmittel mit Sauerstoff übertragender Funktion medizinisch zu verwenden, ist Gegenstand zahlreicher Druckschriften. In diesem Zusammenhang wurde schon vor längerer Zeit gefunden, daß sich Lösungen stromafreien Hämoglobins nicht unmittelbar als Blutersatzstoff eignen, weil nicht-Erythrozyten-gebundenes Hämoglobin relativ schnell in seine Untereinheiten zerfällt und diese Untereinheiten, deren Molekulargewicht kleiner als 64.500 D ist, rasch durch die Nieren ausgeschieden werden. Die intravasale Verweilzeit beträgt ungefähr 100 min.

Um die Dissoziation des Hämoglobins in die Untereinheiten zu verhindern und dadurch die intravasale Halbwertszeit zu vergrößern, wurde versucht, Hämoglobin intramolekular oder intermolekular mit geeigneten bifunktionellen Vernetzungsmitteln zu vernetzen, um eine Molekülgröße sicherzustellen, die eine längere Verweilzeit im Kreislauf garantiert.

So werden in der DE-OS 24 17 619 Plasmaprotein-Ersatzmittel beschrieben, die Hämoglobin enthalten, dessen Untereinheiten mit Hilfe von Dialkyldicarbonsäureimidaten zu Polymerisaten mit mittleren Molekulargewichten zwischen 68.000 und 600.000 vernetzt werden. Eine Vernetzung kann alternativ auch intermolekular zwischen verschiedenen Hämoglobin-Tetrameren oder gleichzeitig sowohl intra- als auch intermolekular erfolgen.

Die DE-OS 26 07 706 offenbart ebenfalls wasserlösliche Hämoglobine, deren Untereinheiten mit Hilfe von geeigneten Vernetzungsmitteln, z.B. Triazinen, mehrfach substituierten mehrfunktionellen Benzol-Derivaten, linearen oder cyclischen mehrfunktionellen Poly-Alkylenderivaten, Dicarbonsäure-Derivaten oder Dialdehyden, zu Polymeren mit Molekulargewichten von 64.000 bis 1.000.000 vernetzt werden. Auch mit derartigen Vernetzungsmitteln wird eine intermolekulare Vernetzung von Hämoglobintetrameren untereinander beschrieben.

Gegenstand der EP-A 81 301 535.1 ist ein Verfahren zur Herstellung künstlicher roter Blut-Zellen, die wäßrige, stromafreie Hämoglobinlösungen in Membranen von polymerisiertem Hämoglobin enthalten. Zur Polymerisation des Hämoglobins und damit zum Schutz gegen raschen Abbau im Körper werden die gleichen Vernetzungsmittel eingesetzt, wie in der genannten DE-OS 26 07 706.

In einem anderen Ansatz zur Erhöhung der intravasalen Halbwertszeit stromafreien Hämoglobins im Organismus wurde versucht, Hämoglobin an physiologisch unbedenkliche hochpolymere Materialien kovalent zu binden. Als physiologisch verträgliche Polymere wurden Hydroxyethylstärke (DE-OS 26 16 086) und weitere über Brücken kovalent gebundene Polysaccharide (DE-OS 30 29 307), Inulin (EP-A 81 302 858.6), Dextran (S. Thamm, J. Blumenstein und J. T. Wong, Proc. Nat. Acad. Sci. USA, 73, 2128 (1976); R. G. Humphries et al, Proceedings of the B.P.S., 191 (1981); J. E. Baldwin et al, Tetrahedron 37, 1723 (1981)) oder Polyethylenglykole unterschiedlicher Molekulargewichte (K. Ajisaka et al, Biochemical and Biophysical Research Communications 97, 1076 (1980); US-PS 4 301 144) beschrieben.

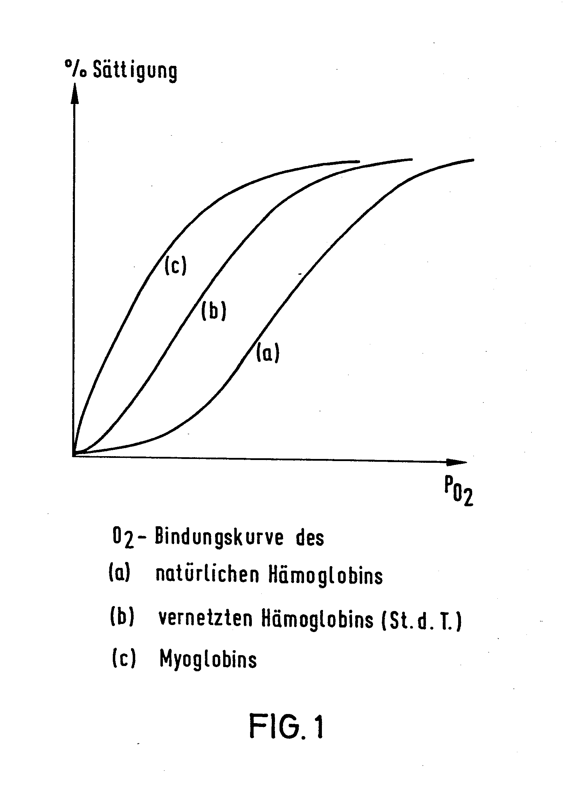

In allen genannten Druckschriften werden hämoglobinhaltige Moleküle mit z.T. hohem Molekulargewicht beschrieben, in denen die kovalente Bindung des Hämoglobins an das Vernetzungsmittel bzw. an das Polymer in unerwünschter Weise die für die Sauerstoff-Transportfunktion unabdingbare natürliche Quartärstruktur des Proteinanteils des Hämoglobins verändert und damit die natürlichen Eigenschaften des Hämoglobins nachteilig beeinflußt. Dies hat zur Folge, daß desoxygeniertes Hämoglobin zwar Sauerstoff bindet, das oxygenierte Hämoglobin den gebundenen Sauerstoff im peripheren Gewebe jedoch nicht mehr ausreichend abgeben kann. Diese Erhöhung der Sauerstoffaffinität zeigt sich meßtechnisch in einer Verschiebung des Mittelteils der Sauerstoffdissoziationskurve nach links; Figur 1 zeigt (a) die Sauerstoffdissoziationskurve des natürlichen Hämoglobins und (b) die entsprechende Kurve bei erhöhter Sauerstoffaffinität gemäß dem Stand der Technik.

Da die Quartärstruktur des natürlichen Hämoglobins durch kovalente Bindungen des Protein-Anteils an Vernetzungsmittel oder Polymermoleküle gestört ist, weisen vernetzte bzw. Polymer-gebundene Hämoglobine ausserdem verminderte Kooperativität bezüglich des Bindungsvermögens für Sauerstoff auf. Quantitatives Maß dieses Effektes ist die Verminderung des Hill-Koeffizienten, der von 2,8 auf geringere Werte absinkt. Meßtechnisch ist dies darin erkennbar, daß sich die für funktionell intaktes Hämoglobin typische sigmoidale Kurvenform (vgl. Fig. 1(a)) für vernetzte bzw. Polymer-gebundene Hämoglobin-Derivate mehr und mehr der Hyperbelform der Kurve des Myoglobins annähert. In Figur 1 zeigt (b) die Kurve für Hämoglobin mit geringer Kooperativität der Untereinheiten und (c) die Kurve des Myoglobins.

Der nachteilige Zusammenhang zwischen einer durch Vernetzung oder Anbindung des Hämoglobins an Polymere herbeigeführten Molekülvergrößerung und einer unerwünschten und für die Verwendung als Blutersatzstoffe nachteiligen Erhöhung der Sauerstoffaffinität läßt sich auch dadurch nicht beseitigen, daß man an die Phosphatbindungsstelle von vernetztem Hämoglobin allosterische Effektoren anbindet und versucht, auf diesem Wege das Sauerstoffabgabevermögen der vernetzten oder polymergebundenen Hämoglobine zu verbessern. So werden in der DE-OS 27 14 252 Verfahren zur Herstellung von mit Dialdehyden vernetzten Hämoglobinen beschrieben, die anstelle des natürlich an die Phosphatbindungsstelle gebundenen allosterischen Modulators 2.3-Diphosphorglycerat Pyridoxal-5-phosphat enthalten.

Dem gleichen Ansatz folgend, wird in der DE-OS 31 44 705 auch Inosithexaphosphat als allosterischer Modulator offenbart. Wenn auch die Einführung des Modulators eine deutliche Verbesserung des Sauerstoffabgabevermögens mit sich bringt, so kommen doch auch bei den allosterisch modifizierten Hämoglobinpräparaten die Nachteile zum Tragen, die aus einer intramolekularen Vernetzung der Hämoglobinuntereinheiten oder der inter- und/oder intramolekularen Vernetzung von Hämoglobin-Tetrameren resultieren. Für einen breiten therapeutischen Einsatz sind auch derartige Präparate noch nicht geeignet.

Aufgabe der vorliegenden Erfindung ist es nun, ein als $O_2$- und $CO_2$-Träger geeignetes und unter natürlichen Bedingungen zur Sauerstoffaufnahme bzw. -abgabe befähigtes hämoglobinhaltiges Blutersatzmittel zur Verfügung zu stellen, das die genannten Nachteile nicht aufweist. Insbesondere sollte vermieden werden, die bindenden Wechselwirkungen der Hämoglobin-Monomeren untereinander durch Vernetzung oder Anbindung an Polymere zu stören und dadurch das kooperative Bindungsverhalten der Hämoglobinmonomeren in Bezug auf ihre Bindung zu molekularem Sauerstoff zu verändern. Andererseits sollte die intravasale Verweildauer möglichst hoch sein und eine rasche Ausscheidung über die Nieren verhindert werden.

Diese Aufgabe wird erfindungsgemäß dadurch gelöst, daß ein hämoglobinhaltiger Blutersatzstoff zur Verfügung gestellt wird, in dem anionische Liganden, die sich mit wasserlöslichen, physiologisch unbedenklichen polymeren Materialien verknüpfen lassen, an die natürliche Phosphatbindungsstelle des Hämoglobins gebunden werden, an der in natürlichem Hämoglobin 2.3-Diphosphoglycerat angelagert ist. Die Sauerstoffaffinität des derart modifizierten Hämoglobins wird somit nicht über Vernetzer oder gebundene Polymermoleküle beeinflußt, sondern über Makromoleküle, die ausschließlich an die natürliche allosterische Bindungsstelle des Hämoglobins gebunden sind.

Gegenstand der vorliegenden Erfindung sind somit Konjugate makromolekularer Verbindungen an Hämoglobin, die als Makromolekül ein Addukt eines oder mehrerer wasserlöslicher, physiologisch unbedenklicher Polymerer an einen anionischen Liganden sowie gereinigtes, von endogenen Liganden isoliertes Humanhämoglobin A enthalten, wobei die Makromoleküle eine den Anforderungen entsprechend variable Molekulargewichtsverteilung von 400 bis 500.000 D aufweisen und nichtkovalent reversibel über die Liganden an das allosterische Bindungszentrum des Hämoglobins gebunden sind.

Die Erfindung umfaßt außerdem ein Verfahren zur Herstellung derartiger Konjugate sowie sie enthaltende Arzneimittel.

Polymere, wasserlösliche physiologisch verträgliche Verbindungen oder deren Derivate, die Bestandteile der erfindungsgemäßen Konjugate sind, enthalten pro Monomereinheit eine oder mehrere polare Gruppen, bevorzugt Hydroxy-, Amino- und/oder Carboxylgruppen. Bevorzugt werden für die erfindungsgemäßen Konjugate natürlich vorkommende Polymere verwendet, es können jedoch auch synthetische Polymere und ihre Derivate eingesetzt werden.

Erfindungsgemäß enthalten die Konjugate makromolekularer Verbindungen an Hämoglobin Polymere aus der Gruppe Polyvinylpyrrolidon und seine Derivate, Dextran und seine Derivate, Polyvinylalkohole, Polysaccharide, lösliche Stärken und deren Derivate, Hydroxyalkylstärken unterschiedlicher Substitutionsgrade, Mucopolysaccharide, Polyethylenglykole und Polypropylenglykole und deren Mischpolymerisate, Proteine und deren Derivate, Tenside, Polyole, Polymethacrylate und Polymethylacrylate, Liposomen und Emulsionen von Fett in Wasser.

Als Polymere sind insbesondere Dextran und seine Derivate oder Polyethylenglykole und deren Derivate geeignet; besonders bevorzugt werden 3-Bromo-2-hydroxypropyldextran, $\alpha$ ,$\omega$ -disubstituierte Derivate des Polyethylenglykols und Diglycidylether des Polyethylenglykols.

Die Molekulargewichte der genannten Polymeren sind über einen weiten Bereich gestreut und können innerhalb der Genzen von 400 bis 500.000 D liegen, wobei Polymere mit Molekulargewichten von 400 bis 50.000 D bevorzugt sind.

Erfindungsgemäß werden Polymere der genannten Gruppe mit solchen niedermolekularen Substanzen chemisch verknüpft, die sich über nichtkovalente Bindungen an die Phosphatbindungsstelle des Hämoglobins anlagern können. Diese Liganden erlauben eine Steuerung der Sauerstoffaffinität des Hämoglobins.

Als polymergebundene Ligandmoleküle, die meist in anionischer Form vorliegen, kommen außer den natürlich an Hämoglobin gebundenen Liganden Zuckerphosphate und deren Derivate, Inositphosphate und deren Derivate, Inositsulfate und deren Derivate, Nukleotidphosphate und deren Derivate, Pyridoxalphosphat bzw. -sulfat und deren Derivate, Mucopolysaccharide und deren Derivate, ß-Naphtholphosphate, Salicylsäure bzw. p-Hydroxybenzoesäure und deren Derivate, aromatische Aldehyde und deren Derivate, und Benzolsulfonsäuren sowie deren Derivate in Frage. Bevorzugte Ligandenmoleküle sind Zuckerphosphate, Nukleotidphosphate, Pyridoxalphosphat oder Inositphosphate.

Als besonders bevorzugter Ligand wird Inosithexaphosphat angesehen, da es zu Hämoglobin eine um mehr als den Faktor $10^3$ festere Bindung ausbildet als der im intakten Erythrozyten enthaltene Modulator 2.3-Diphosphoglycerat.

Wo erforderlich, können Polymermolekül und Ligand auch über einen Spacer miteinander verknüpft sein. Als Spacer finden solche Moleküle Verwendung, die an zwei Enden je eine zu einer Kopplung geeignete funktionelle Gruppe tragen und die es erlauben, zwischen dem Liganden und dem Polymermolekül eine bestimmte Entfernung kontrolliert einzustellen. Als Spacermoleküle sind insbesondere Dialdehyde sowie Dicarbonsäuren und deren Derivate geeignet, deren funktionelle Gruppen durch Alkylenbrücken mit 1 bis 4 C-Atomen verbunden sind. Bevorzugter Spacer gemäß der vorliegenden Erfindung ist Glutardialdehyd.

Die aus einem wasserlöslichen, ungeladenen, physiologisch unbedenklichen Polymeren und einem der genannten anionischen Liganden aufgebauten Makromoleküle reagieren spontan mit isoliertem, stromafreiem, vorzugsweise desoxygeniertem Hämoglobin, wobei sich über nichtkovalente Bindungen zwischen dem Liganden und der Phosphatbindungsstelle des Hämoglobins zusammengehaltene Polymer-Ligand-Hämoglobin-Konjugate hoher Bindungskonstante und Stabilität bilden.

Diese Komplexe sind in der Lage, Sauerstoff reversibel zu binden und wieder abzugeben, wobei eine Erniedrigung der $O_2$-Affinität des mit dem Addukt modulierten Hämoglobins beobachtet wird. Dies wird meßtechnisch sichtbar in einer Verschiebung des Mittelteils der Sauerstoffdissoziationskurve nach rechts, d.h. zu höheren $O_2$-Partialdrucken hin. Die Kooperativität der Hämoglobin-Untereinheiten bei der Sauerstoffaufnahme bzw. -abgabe bleibt erhalten, was sich in einem sigmoidalen Verlauf der Sauerstoffdissoziationskurve zeigt.

Die erfindungsgemäßen Konjugate makromolekularer Verbindungen an Hämoglobin zeichnen sich also dadurch aus, daß unter Verzicht auf Kopplungs- oder Vernetzungsreaktionen ein Hämoglobin enthaltendes Blutersatzmittel oder Blutplasma-Streckmittel mit sauerstoffübertragender Funktion zur Verfügung gestellt wird, das aufgrund eines erhöhten Molekulargewichts eine höhere intravasale Verweilzeit aufweist, molekularen Sauerstoff reversibel binden und aufgrund einer relativ niedrigen $O_2$-Affinität auch leicht wieder abgeben kann, ohne daß zur Erreichung dieser Effekte die Hämoglobin-Tetramerstruktur durch Vernetzung oder Komplexbildung wesentlich gestört würde.

Die Eigenschaften derartiger Hämoglobin-Konjugate sind durch die Wahl zahlreicher Parameter in weiten Grenzen variierbar. So können die chemische Natur und die Eigenschaften des verwendeten Polymeren, z.B. dessen Molekulargewichtsverteilung, Löslichkeit oder Vernetzungsgrad, verändert und damit die Verweilzeit im Kreislauf gesteuert werden. Außerdem kann die Festigkeit der Bindung des Polymer-Ligand-Adduktes an Hämoglobin durch die Anzahl der geladenen Gruppen (Phosphat-Gruppen) am Liganden beeinflußt werden.

Die Konzentration des Liganden, d.h. der Grad der Beladung des Polymeren mit dem Liganden, hat Auswirkungen auf die Menge an Hämoglobin, die in nichtkovalenter Form maximal gebunden werden kann. Über die Ligandenkonzentration ist also ein direkter Einfluß auf die Sauerstoff-Transportkapazität des als Blutersatzmittel eingesetzten Hämoglobins möglich.

Konjugate mit unterschiedlichen Eigenschaften werden ebenfalls dadurch erhalten, daß man den Abstand zwischen Ligand und Polymermolekül sowie die Natur der Bindung, mit der die beiden Moleküle verknüpft werden, variiert. Dabei sind eine direkte Verknüpfung oder die Verknüpfung über einen Spacer möglich, wobei auch die Natur der Bindung (Etherbindung, Phosphatsäurediesterbindung usw.) die Eigenschaften der gebildeten Konjugate beeinflussen.

Schließlich können auch an jedem Polymermolekül mehrere Liganden (mit oder ohne Spacer) und damit auch mehrere Hämoglobinmoleküle gebunden sein. Als Möglichkeiten sind je ein Liganden-Hämoglobin-Konjugat an den beiden Enden eines Polymermoleküls oder auch mehrere Liganden-Hämoglobin-Konjugate an Seitenketten eines verzweigten Polymeren möglich.

Die Erfindung umfaßt ebenfalls zwei- oder mehrphasige Polymer-Ligand-Hämoglobin-Systeme, in denen ein festes, unlösliches Polymermolekül an der Oberfläche, gegebenenfalls unter Zwischenschaltung eines Spacers, mehrere Ligandenmoleküle und damit auch mehrere Hämoglobinmoleküle gebunden enthält. Beispiele dafür sind Liposomen oder Emulsionen, an die in der nichtwässrigen Phase die Ligand-Hämoglobin-Konjugate gebunden sind. Die Liganden wie auch Hämoglobin liegen dabei in der wässrigen Phase vor, wobei die Liganden über die nichtwässrige Phase verknüpft sind.

Selbstverständlich ist es ebenfalls im Rahmen der vorliegenden Erfindung möglich, Hämoglobin zusätzlich mit den üblichen bi- oder polyfunktionellen Vernetzungsmitteln zu modifizieren. Im Zuge dieser Maßnahme verändern sich jedoch die Eigenschaften der Hämoglobin-Lösungen in der aus dem Stand der Technik bekannten nachteiligen Weise, d.h. die Sauerstoffaffinität ist im Vergleich zu dem unvernetzten Präparat deutlich erhöht und die Kooperativität der Hämoglobin-Untereinheiten bezüglich ihres Bindungsvermögens für molekularen Sauerstoff ist vermindert. Die Konjugatbildung zwischen Polymermolekül, modulierenden Liganden und Hämoglobin führt jedoch zu einer so deutlichen Erniedrigung der Sauerstoffaffinität und Wiederherstellung der Kooperativität der Untereinheiten, daß erfindungsgemäß modifizierte, aber vernetztes Hämoglobin enthaltende Konjugate den aus dem Stand der Technik bekannten ausschließlich vernetzten hämoglobinhaltigen Blutersatzstoffen deutlich überlegen sind.

Zur Herstellung der erfindungsgemäßen Konjugate makromolekularer Verbindungen an Hämoglobin werden primär die Polymeren synthetisiert oder kommerziell erhältliche Polymere nach an sich bekannten Verfahren chemisch modifiziert. So können beispielsweise unmodifizierte Polyethylenglykole oder auch ihre Glycidylether oder ihre $\alpha$ ,$\omega$ -diaminosubstituierten Derivate verwendet werden.

Die gemäß der Erfindung verwendbaren Polymere werden unter kontrollierten Bedingungen (pH-Wert, Temperatur, Konzentration) in wässrigen Lösungen, gegebenenfalls unter Verwendung geeigneter Puffer, mit dem jeweiligen Liganden umgesetzt. Als Puffer werden bevorzugt Natriumboratpuffer (pH 9,0) oder Phosphatpuffer (pH 7,2) verwendet. Es sind jedoch auch andere dem Fachmann bekannte Puffersysteme verwendbar.

Die Umsetzung des Polymermoleküls mit dem Liganden kann, sofern gewünscht, unter Zugabe eines geeigneten bifunktionellen Moleküls als Spacer geschehen. Endprodukt sind physiologisch verträgliche, wasserlösliche

Makromeküle mit Molekulargewichten im Bereich von 400 bis 500.000 D, an die endständig meist phosphathaltige Liganden gebunden sind.

Die resultierenden anionischen Addukte der Polymermoleküle an anionische Liganden werden nachfolgend nach an sich bekannten Methoden, z.B. durch Diafiltration gegen Wasser, gereinigt, wobei über die Wahl einer bestimmten Ausschlußgrenze der Filtrationsmembran ein den Anforderungen entsprechendes Molekulargewicht eingestellt werden kann.

Die Isolierung funktionell intakten Hämoglobins (HbA) aus humanem Vollblut in größerem Maßstab ist beispielsweise in "Methods in Enzymology, Band 76, Seite 97 ff" beschrieben. Das nach dem beschriebenen Verfahren gewonnene, gereinigte und von endogenen Liganden befreite (stripped) Hämoglobin wird in einer wässrigen Pufferlösung, bevorzugt in Phosphatpuffer (pH 7,4) aufgeschlämmt und durch mehrmaliges Evakuieren der Lösung und anschließendes Spülen mit gereinigtem Stickstoff desoxygeniert.

Die resultierende hämoglobinhaltige Lösung wird mit wässrigen, gegebenenfalls gepufferten Lösungen der polymeren ligandtragenden Substanzen bei pH-Werten zwischen 5,0 und 8,0 vermischt. Bevorzugt wird im sauren pH-Bereich gearbeitet. Dabei bilden sich spontan über nichtkovalente Bindungen gebundene, stabile Konjugate der makromolekularen Verbindungen an Hämoglobin.

Die erfindungsgemäß hergestellten gepufferten Lösungen von Konjugaten makromolekularer Verbindungen an Hämoglobin weisen gegenüber vernetzten Hämoglobinen eine deutlich verbesserte Sauerstoffaffinität auf. So wurden bei Funktionsuntersuchungen Werte für den Sauerstoffpartialdruck bei 50%iger Sättigung des an die makromolekularen Addukte gebundenen Hämoglobins ($P_{50}$) gefunden, die mit denen des natürlichen, erythrozytengebundenen Hämoglobins vergleichbar sind oder diese sogar übertreffen.

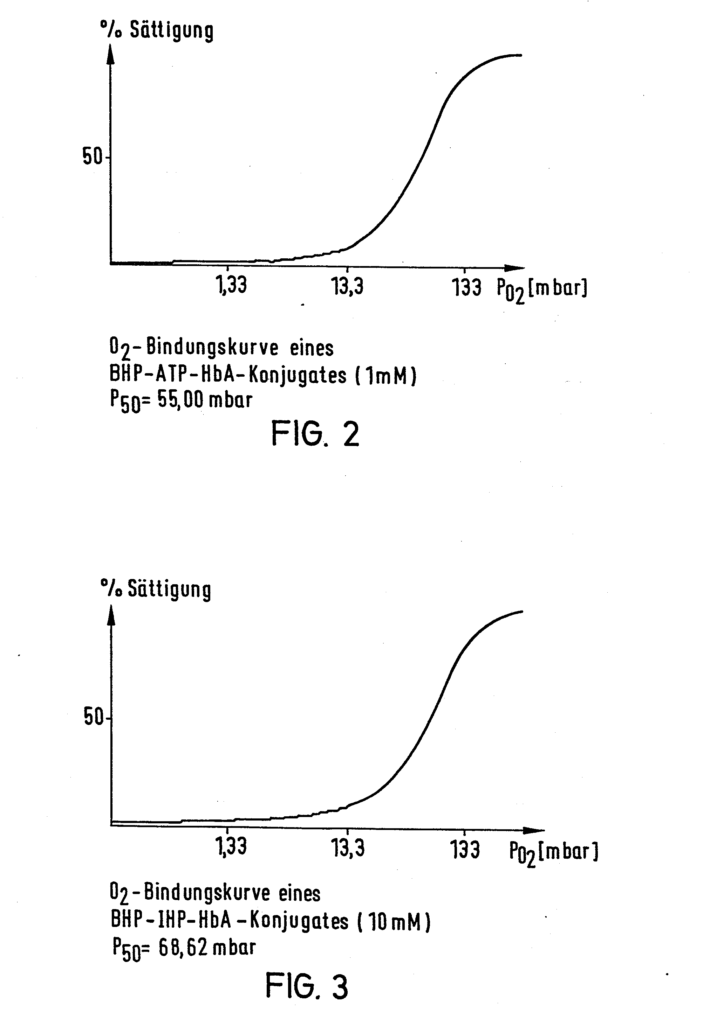

Wie aus den Figuren 2 bis 5 zu ersehen ist, weisen die erfindungsgemäßen Konjugate ebenfalls eine hohe Kooperativität der Hämoglobinuntereinheiten bezüglich der Bindung bzw. Freisetzung molekularen Sauerstoffs auf. Dies zeigt sich in den Figuren an der sigmoidalen Form der Sauerstoffbindungskurve und wird quantitativ an einem Hill-Koeffizienten nahe dem natürlichen Wert sichtbar.

Durch die Konjugatbildung wird ebenfalls die intravasale Verweilzeit des Hämoglobins gegenüber stromafreiem Hämoglobin wesentlich erhöht. Während sie für freies, unmodifiziertes Hämoglobin bei ungefähr 85 bis 100 min liegt (J.E. Baldwin et al, Med. Lab. Sci. <u>39</u>, 45 (1982)), wurden für die erfindungsgemäßen Konjugate Verweilzeiten von bis zu mehreren Tagen gemessen.

Auch die sonstigen Eigenschaften (kolloidosmotischer Druck, rheologische Eigenschaften, Stabilität, Lagerfähigkeit) machen die erfindungsgemäßen Konjugate hervorragend geeignet für die Verwendung als Blutersatzstoff oder Blutplasma-Streckmittel mit sauerstoffübertragender Funktion.

Die für die Verabreichung der erfindungsgemässen Konjugate notwendigen Hilfs- und Zusatzstoffe, wie z.B. Ringer-Lactat-Lösung o.ä., sind dem Fachmann grundsätzlich bekannt und bedürfen keiner weiteren Erläuterung.

Die Erfindung wird durch die nachstehenden Beispiele näher erläutert.

## Beispiel 1

### Synthese eines Konjugates von Adenosin-5-triphosphat (ATP) an BHP-Dextran 70

1 g lyophilisiertes BHP-Dextran (3-Bromo-2-hydroxypropyldextran, Hersteller: Pharmacia Fine Chemcial AB, Uppsala) wurde in 5 ml 0,1 M Natriumboratpuffer, pH 9,0, bei Raumtemperatur gelöst und der pH-Wert mit 0,5 M NaOH auf pH 11,0 eingestellt und 15 Minuten beibehalten. Man setzte 500 µmole Adenosintriphosphat (ATP) zu, das in 5 ml des gleichen Puffers gelöst war, korrigierte den pH-Wert mit festem $NaHCO_3$ auf pH 10,0 und inkubierte unter Rühren weitere 24 Stunden bei Raumtemperatur. Nicht umgesetzte aktivierte Gruppen wurden mit 0,1 M wässriger Glycerinlösung 10 Stunden gerührt und dadurch blockiert und das Reaktionsgemisch durch Diafiltration gegen Wasser über eine Amicon H 1 P 10-Hohlfasermembran (Ausschlußgrenze: 10.000) gereinigt. Das Retentat wurde lyophilisiert. Das weiße Produkt (0,8 g) wies einen ATP-Gehalt von 0,18 m mole/g auf.

## Beispiel 2

### Synthese eines Kopplungsproduktes von Inositolhexaphosphat-Natriumsalz (IHP) am BHP-Dextran 70

Anstelle des in Beispiel 1 eingesetzten ATP wurden 5 mM Inositolhexaphosphat-Natriumsalz (Sigma) mit 1 g BHP-Dextran umgesetzt und aufgearbeitet. Man erhielt ein weißes Produkt (0,6 g) mit einem IHP-Gehalt von 0,095 mmol/g.

## Beispiel 3

Die gemäß Beispiel 1 und 2 hergestellten Produkte wurden auf eine Konzentration von entsprechend 1 mM ATP bzw. 10 mM IHP in 100 ml 0,1 M Phosphatpuffer pH 7,3 gelöst und mit desoxygeniertem, nach "Methods in Enzymology, Band 76, S. 97" ff hergestelltem Humanhämoglobin (HbA) vermischt, so daß eine Endkonzentration des HbA von 6% erreicht wurde.

Die Sauerstoffbindungskurven dieser Mischungen, gemessen bei pH 7,4 und 37°C in BISTRIS-Puffer zeigen Fig. 2 und Fig. 3. Die $P_{50}$-Werte dieser Präparate lagen bei:

| Addukt aus Beisp. | Probe | Konzentration | $P_{50}$ $/\overline{\text{mbar}}\_7$ | Sauerstoffbindungskurve in |
|---|---|---|---|---|
| 1 | BHP-ATP | 1mM | 55,00 | Fig. 2 |
| 2 | BHP-IHP | 10mM | 68,62 | Fig. 3 |

## Beispiel 4

Die ATP bzw. IHP-Konzentration der dextrangebundenen Liganden wurde in einem zu Beispiel 3 analogen Experiment auf folgende Werte eingestellt:

| Addukt aus Bsp. | Probe | Konzentration | $P_{50}$ $/\overline{\text{mbar}}\_7$ | Sauerstoffbindungskurve in |
|---|---|---|---|---|
| 1 | BHP-ATP | 0,2 mM | 21,01 | Fig. 4 |
| 2 | BHP-IHP | 1,0 mM | 30,15 | Fig. 5 |

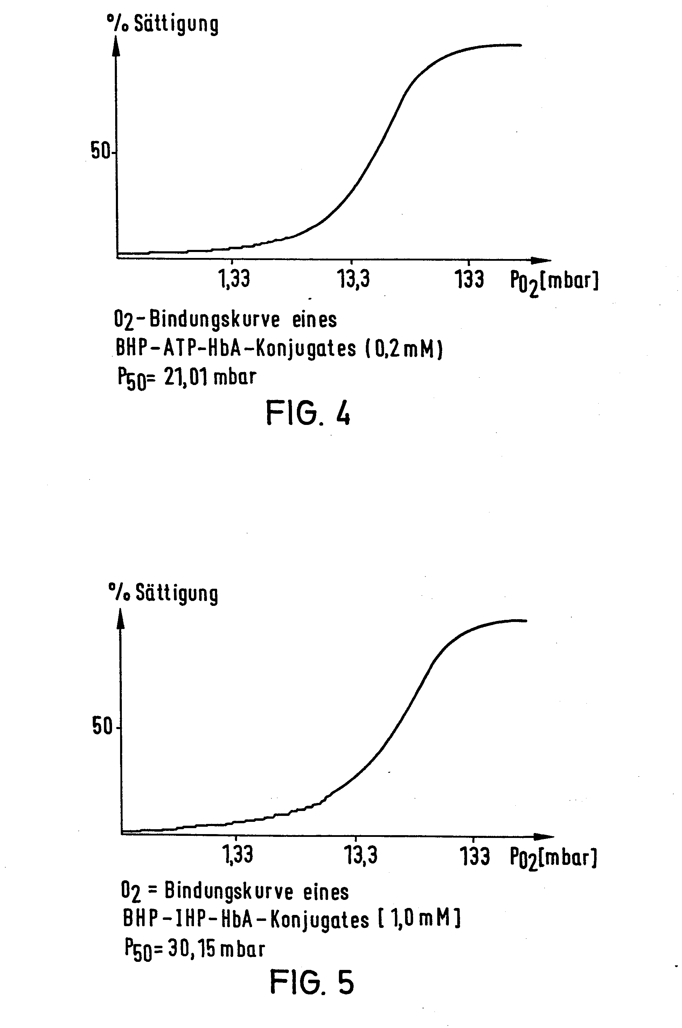

Die Figuren 4 und 5 zeigen die zugehörigen Sauerstoffbindungskurven, gemessen bei pH 7,4 und 37°C in BISTRIS-Puffer.

## Beispiel 5

200 ml einer 18%igen HbA-Lösung, gelöst in 0,1 m Phosphatpuffer, pH 7,4, wurden durch mehrmaliges Evakuieren und Spülen mit Stickstoff desoxygeniert. Man setzte 1,0 g des gemäß Beispiel 2 hergestellten BHP-IHP-Konjugates zu, stellte den pH-Wert auf 6,8 ein und gab unter Schutzgas 7,2 ml einer 5%igen Glutardialdehydlösung zu, rührte 30 Minuten und reduzierte das Produkt durch Zugabe von 0,4 g $NaBH_4$. Die Reaktionsmischung wurde über eine Ultrafiltrationsmembran mit Ausschlußgrenze 10.000 (Amicon H1P10-Hollowfiber) unter ständiger Zugabe von 0,1 M Phosphatpuffer, pH 7,4 über 6 Stunden diafiltriert und die Mischung auf eine Hämoglobinkonzentration von 6% eingestellt. Der Halbsättigungsdruck dieses Präparates lag bei: $P_{50}$ = 47,9 mbar.

## Beispiel 6

Gemäß den Angaben von S.G. Cohan und H.C. Haas (JACS 75, 1733 (1953)) wurde 1 mmol Polyethylenglycol 10.000 (Merck) mit 20 mg Zinn-IV-Chlorid in 200 ml Xylol mit 2 mmol Epichlorhydrin umgesetzt und unter Weglassung des fraktionierten Destillationsschrittes aufgearbeitet. Man erhielt 9,2 g Diglycidylether des Polyethylenglykols als Produkt.

1 g (0,2 mmol) des Materials wurde unter den in Beispiel 1 beschriebenen Bedingungen in Boratpuffer mit 0,5 mmol ATP 10 Stunden bei Raumtemperatur umgesetzt. Das Produkt wurde durch 6-stündige Diafiltration mit Wasser über eine Hohlfaser-Ultrafiltrationsmembran (H 1 P 5, Amicon) gereinigt und lyophilisiert. Wie in Beispiel 3 beschrieben, wurden 10 mmol des Adduktes ATP-PEG mit desoxygeniertem HbA der Endkonzentration 6% vermischt und die $O_2$-Bindungskurve registriert. Der $P_{50}$-Wert betrug 48,15 mbar.

## Beispiel 7

Gemäß der von W. Kern et al in Makromol. Chem. <u>180</u>, 2539-2542 (1979) angegebenen Vorschrift wurde zunächst aus Polyethylenglykol 400 (Merck) über das $\alpha$, $\omega$-Ditosylat und Umsetzung mit Ethanolamin das $\alpha$, $\omega$-Diaminosubstituierte Polyethylenglykol hergestellt.

40 g (10 mmol) des $\alpha$, $\omega$-Diaminopolyethylenglykols wurden in 1000 ml 0,1 M Phosphatpuffer, pH 7,2 gelöst und 8,0 g (30 mmol) Pyridoxalphosphat unter Rühren zugegeben. Man ließ über Nacht bei Raumtemperatur reagieren und reduzierte 2 Stunden mit 1,9 g $NaBH_4$ bei Raumtemperatur. Die Reinigung des Produktes erfolgte auch in diesem Falle durch Dialfiltration mit Wasser über eine Hohlfasermembran H 1 P 1 (Amicon, Ausschlußgrenze: 1.000), bis im Ultrafiltrat spektralphotometrisch kein Pyridoxalphosphat mehr nachweisbar war. Das Produkt wurde lyophilisiert (Ausbeute 73%). Die gemäß Beispiel 3 durchgeführte Umsetzung mit desoxyeniertem HbA ergab ein Produkt mit einer Hb-Konzentration von 6% und einer PEG-(Pyridoxalphosphat)$_2$-Konzentration von 2,5 mM (10 g/Liter), das einen $P_{50}$-Wert von 54,53 mbar aufwies.

**P a t e n t a n s p r ü c h e**

1. Konjugate makromolekularer Verbindungen an Hämoglobin, enthaltend

A) als Makromolekül ein Addukt eines oder mehrerer wasserlöslicher, physiologisch unbedenklicher Polymerer aus der Gruppe Polyvinylpyrrolidon und seine Derivate, Dextran und seine Derivate, Polyvinylalkohole, Polysaccharide, lösliche Stärken und deren Derivate, Hydroxyalkylstärken unterschiedlicher Substitutionsgrade, Mucopolysaccharide, Polyethylenglykole und Polypropylenglykole und deren Mischpolymerisate, Proteine und deren Derivate, Tenside, Polyole, Polymethacrylate und Polymethylacrylate, Liposomen und Emulsionen von Fetten in Wasser, an

B) einen anionischen Liganden aus der Gruppe Zuckerphosphate und deren Derivate, Inositphosphate und deren Derivate, Inositsulfate und deren Derivate, Nukleotidphosphate und deren Derivate, Pyridoxalphosphat bzw. -sulfat und deren Derivate, Mucopolysaccharide und deren Derivate, ß-Naphtholphosphate, Salicylsäure bzw. p-Hydroxybenzoesäure und deren Derivate, aromatische Aldehyde und deren Derivate und Benzolsulfonsäuren und deren Derivate, sowie

C) gereinigtes, von endogenen Liganden isoliertes Human-Hämoglobin A,

wobei die Makromoleküle eine den Anforderungen entsprechend variable Molekulargewichtsverteilung von 400 bis 500.000 D aufweisen und nichtkovalent reversibel über die Liganden an das allosterische Bindungszentrum des Hämoglobins gebunden sind.

2. Konjugate nach Anspruch 1, dadurch gekennzeichnet, daß sie als Polymere Dextran und seine Derivate oder Polyethylenglykole und deren Derivate enthalten.

3. Konjugate nach Ansprüchen 1 und 2, dadurch gekennzeichnet, daß sie als Polymermoleküle 3-Bromo-2-hydroxypropyldextran, $\alpha$,$\omega$-disubstituierte Derivate des Polyethylenglykols oder Diglycidylether des Polyethylenglykols enthalten.

4. Konjugate nach Ansprüchen 1 bis 3, dadurch gekennzeichnet, daß die Polymeren eine Molekulargewichtsverteilung von 400 bis 50.000 D aufweisen.

5. Konjugate nach Ansprüchen 1 bis 4, dadurch gekennzeichnet, daß sie als an das Polymermolekül gebundene anionische Liganden Zuckerphosphate und deren Derivate, Nukleotidphosphate und deren Derivate, Pyridoxalphosphat und deren Derivate, Inositphosphate und deren Derivate, Acetylsalicylsäure und deren Derivate, oder Benzolsulfonsäuren und deren Derivate enthalten.

6. Konjugate nach Ansprüchen 1 bis 5, dadurch gekennzeichnet, daß sie als an das Polymermolekül gebundene anionische Liganden Inosithexaphosphat-Natrium, Adenosintriphosphat, Fructose-1.6-diphosphat, Pyridoxalphosphat oder 4-Isothiocyanat-benzolsulfonsäure enthalten.

7. Konjugate nach Ansprüchen 1 bis 6, dadurch gekennzeichnet, daß im Makromolekül die Bindung zwischen Ligand und Polymermolekül über einen Spacer erfolgt.

8. Konjugate nach Ansprüchen 1 bis 7, dadurch gekennzeichnet, daß sie gereinigtes, von endogenen Liganden isoliertes, desoxygeniertes Human-Hämoglobin A enthalten.

9. Konjugate nach Ansprüchen 1 - 8, dadurch gekennzeichnet, daß sie gegebenenfalls mit bi- und/oder polyfunktionellen Vernetzungsmitteln modifiziertes Hämoglobin enthalten.

10. Verfahren zur Herstellung von Konjugaten makromolekularer Verbindungen an Hämoglobin, dadurch gekennzeichnet, daß man bei Raumtemperatur in wässriger Lösung

a) ein Polymeres aus der Gruppe Polyvinylpyrrolidon und seine Derivate, Dextran und seine Derivate, Polyvinylalkohole, Polysaccharide, lösliche Stärken und deren Derivate, Hydroxyalkylstärken unterschiedlicher Substitutionsgrade, Mucopolysaccharide, Polyethylenglykole und Polypropylenglykole und deren Mischpolymerisate, Proteine und deren Derivate, Tenside, Polyole, Polymethacrylate und Polymethylacrylate, Liposomen und Emulsionen von Fetten in Wasser, an

b) einen anionischen Liganden aus der Gruppe Zuckerphosphate und deren Derivate, Inositphosphate und deren Derivate, Inositsulfate und deren Derivate, Nukleotidphosphate und deren Derivate, Pyridoxalphosphat bzw. -sulfat und deren Derivate, Mucopolysaccharide und deren Derivate, ß-Naphtolphosphate, Salicylsäure bzw. p-Hydroxybenzoesäure und deren Derivate, aromatische Aldehyde und deren Derivate und Benzolsulfonsäuren und deren Derivate, addiert,

c) das so gewonnene Addukt nach an sich bekannten Methoden reinigt und

d) mit gereinigtem, von endogenen Liganden isoliertem Human-Hämoglobin A vermischt.

11. Verfahren nach Anspruch 10, dadurch gekennzeichnet, daß man gereinigtes, von endogenen Liganden isoliertes, desoxygeniertes Human-Hämoglobin A verwendet.

12. Verfahren nach Ansprüchen 10 und 11, dadurch gekennzeichnet, daß man zur Reinigung die wässrigen Lösungen der Addukte diafiltriert.

13. Verfahren nach Ansprüchen 10 - 12, dadurch gekennzeichnet, daß man in gepufferter wässriger Lösung arbeitet.

14. Verfahren nach Ansprüchen 10 - 13, dadurch gekennzeichnet, daß man bei einem pH-Wert im Bereich von 5,0 bis 8,0, bevorzugt im sauren Bereich, arbeitet.

15. Arzneimittel, enthaltend Konjugate makromolekularer Verbindungen an Hämoglobin neben für Blutersatzstoffe oder Blutplasma-Streckmittel üblichen Träger- und Hilfsstoffen.

O₂- Bindungskurve des

(a)   natürlichen Hämoglobins

(b)   vernetzten Hämoglobins (St. d. T.)

(c)   Myoglobins

FIG. 1

O$_2$-Bindungskurve eines
BHP-ATP-HbA-Konjugates (1mM)
P$_{50}$= 55,00 mbar

## FIG. 2

O$_2$-Bindungskurve eines
BHP-IHP-HbA-Konjugates (10mM)
P$_{50}$= 68,62 mbar

## FIG. 3

% Sättigung

1,33    13,3    133    $P_{O_2}$[mbar]

$O_2$-Bindungskurve eines
BHP-ATP-HbA-Konjugates (0,2 mM)
$P_{50}$= 21,01 mbar

FIG. 4

% Sättigung

1,33    13,3    133    $P_{O_2}$[mbar]

$O_2$ = Bindungskurve eines
BHP-IHP-HbA-Konjugates [1,0 mM]
$P_{50}$= 30,15 mbar

FIG. 5